# FASCICULE DE BREVET EUROPEEN

(11) **EP 0 424 232 B1**
(45) Date de publication et mention de la délivrance du brevet: **21.02.1996**
(21) Numéro de dépôt: 90402879.2
(22) Date de dépôt: 16.10.1990
(51) Int. Cl.: C07J 9/00

(54) **Procédé de préparation de l'acide**
Verfahren zur Herstellung von Chenodesoxycholsäuren
Process for the production of chenodesoxycholic

(30) Priorité: 17.10.1989 FR 8913569; 17.10.1989 FR 8913570; 17.10.1989 FR 8913571; 17.10.1989 FR 8913572
(43) Date de publication de la demande: 24.04.1991
(73) Titulaire: SANOFI, F-75008 Paris (FR)
(72) Inventeur: Arosio, Roberto, I-22040 Civate (Como) (IT); Rossetti, Vittorio, I-20142 Milano (IT); Beratto, Stefano, I-20123 Milano (IT); Talamona, Armando, I-21100 Varese (IT); Crisafulli, Emilio, I-20146 Milano (IT)
(74) Mandataire: Gillard, Marie-Louise

(56) Documents cités:
- EP-A- 0 063 106
- EP-A- 0 072 293
- EP-A- 0 088 637
- EP-A- 0 230 085
- FR-A- 2 510 583
- US-A- 4 360 470
- US-A- 4 425 273
- JOURNAL OF THE AMERICAN CHEMICAL SOCIETY. vol. 72, 1950, GASTON, PA US pages 5530 - 5536; L. F. FIESER ET AL: 'Oxidation of Steroids. III. Selective Oxidations and Acylations in the Bile Acid Series'

## Description

La présente invention concerne un procédé amélioré de préparation de l'acide chénodésoxycholique (ou acide 3α, 7α-dihydroxy-5β-cholanique).

Des nombreuses synthèses des acides chénodésoxycholique et ursodésoxycholique ont été décrites dans la littérature. On peut citer, par exemple, les synthèses décrites dans les brevets américains US 3891681, US 3945562 et US 4425273, les demandes de brevet Japonais non examinées J50088856 (Derwent 11665W9), J54079260 (Derwent 57301B), et J59039900 (Derwent 84-092393), les demandes de brevet Japonais examinées J75012434 (Derwent 38598W) , J81014120 (Derwent 71422A), et J87054439 (Derwent 38484E), les brevets tchécoslovaques CS-186067 (Chem. Abst. 100, 175130c), et CS-233418 (Chem. Abst. 106, 102613b), les brevets anglais GB-2202850 et GB-2076823, le brevet français FR-1372109, la demande de brevet hollandais NL-7908972, les brevets espagnols ES-489661 (Chem. Abst. 96, 20375m) et ES-499525 (Chem. Abst. 97, 145149t), et les brevets européens EP-63106 et EP-88637.

En effet tous les procédés qui sont utilisés aujourd'hui au niveau industriel pour la préparation de l'acide chénodésoxycholique se placent dans le même schema réactionnel qui comprend substantiellement les étapes suivantes :
- **étape (a)** préparation d'un ester alkylique de l'acide 3α,7α-diacétoxy-12α-hydroxy-5β-cholanique de formule (I) 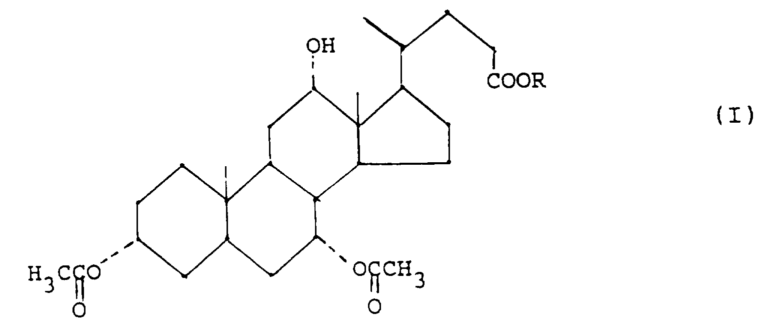 dans laquelle R est méthyle, éthyle, ou n-propyle,
- **étape (b)** conversion d'un composé de formule (I) en un dérivé 12-oxo de formule (II) 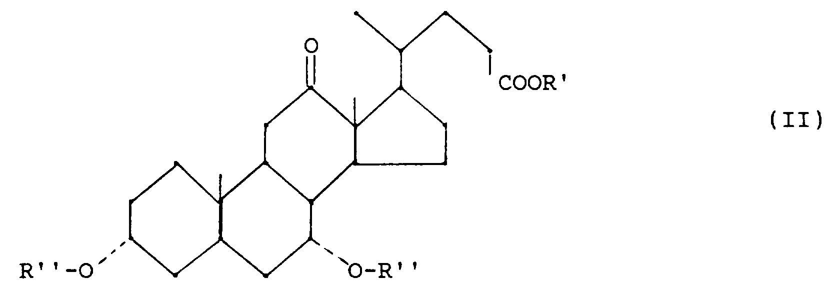 dans laquelle R' et R'' représentent l'hydrogène ou bien R'' représente un groupe acétyle et R' a la même signification que R ci-dessus, et
- **étape (c)** réduction d'un composé de formule (II) en acide chénodésoxycholique de formule (III)

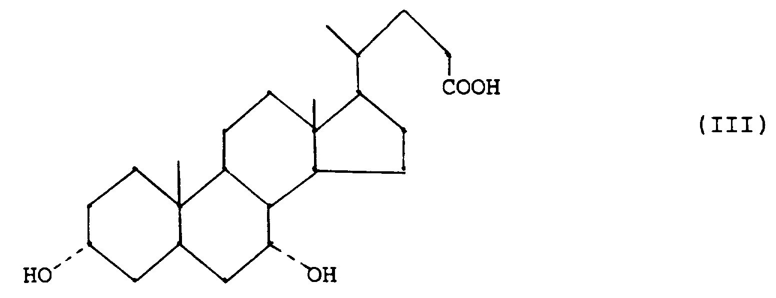

Chacun de ces passages est bien connu mais, dans les formes de réalisation décrites en littérature, présente des inconvenients qui posent des problèmes serieux du point de vue industriel.

Plus particulièrement, en ce qui concerne l'étape a), il est bien connu que les esters de l'acide cholique peuvent être diacétylés en positions 3 et 7, pour obtenir les esters correspondants de l'acide 3α,7α-diacétoxy-12α-hydroxycholanique.

Selon les méthodes classiques, cette diacétylation est conduite notamment par estérification avec l'anhydride acétique dans la pyridine, en utilisant un excès tant d'anhydride acétique que de pyridine et, notamment, en utilisant ces deux derniers produits en tant que solvants de réaction.

Ces méthodes comportent l'utilisation de quantités de réactifs très importantes et la formation d'une quantité non négligeable de sous-produit tri-acétoxylé. Pour éviter la formation de quantités trop importantes de ce sous-produit, il est nécessaire de surveiller le déroulement de la réaction en tenant sous contrôle soit la température,soit la durée de la réaction pour l'arrêter au moment opportun.

L'oxydation d'hydroxycholanates prévue à l'étape (b), généralement réalisée au moyen d'un hypochlorite alcalin et de l'acide acétique, est également bien connue dans la littérature, cette réaction étant conduite en utilisant un excès d'acide acétique. L'inconvenient principal de cette réaction est celui de la libération du chlore comportant, entre autres, des problèmes de contrôle pour éviter des dommages écologiques.

La réduction du composé de formule (II) en acide chénodésoxycholique (III), bien connue comme réduction de Wolff-Kishner modifiée par Huang-Minlon, a été décrite, dans le cadre de la préparation de l'acide chénodésoxycholique, par L.F. Fieser et al., J. Am. Chem. Soc. 1950, 72**,** 5530-5536 et par E. Hauser et al., Helv. Chim. Acta 1960, 43, 1595-1600 et est la méthode couramment utilisée dans le cadre de la fabrication de l'acide chénodésoxycholique.

De toute façon, cette réaction ne donne pas de bons rendements en produit final et la raison principale est due au fait que, selon les méthodes classiques, la réaction doit être conduite ou complétée à température très élevée, ce qui provoque la formation de quantités non négligeables de sous-produits.

On a maintenant trouvé qu'on peut améliorer chacune de ces étapes, en surmontant les problèmes posés par les méthodes connues.

En ce qui concerne la diacétylation en 3 et en 7 d'esters de l'acide cholique, prévue à l'étape (a), on a maintenant trouvé que, de façon générale, la diacétylation en 3 et 7 s'effectue mieux si l'ester de départ ne contient pas de traces de l'alcool estérifiant le cholate d'alkyle et que l'élimination de l'alcool peut être faite par lavage à l'eau d'une solution de cholate d'alkyle et distillation azéotropique.

On a également trouvé qu'on peut effectuer une diacétylation sélective des esters de l'acide cholique en utilisant une quantité stoechiométrique d'anhydride acétique si l'on opère en présence de quantités catalytiques d'une N,N-dialkylaniline ou d'une 4-dialkylaminopyridine et d'une quantité d'amine tertiaire inférieure à une mole d'amine par mole de produit de départ.

Ainsi, la préparation de 3α,7α-diacétoxy-12α-hydroxy-5β-cholanates d'alkyle de formule (I) 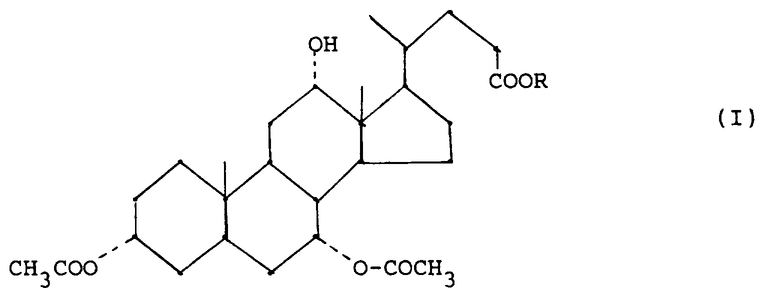 dans laquelle R représente méthyle, éthyle ou n-propyle, est convenablement effectuée par acétylation des esters correspondants de l'acide cholique de formule (VI) 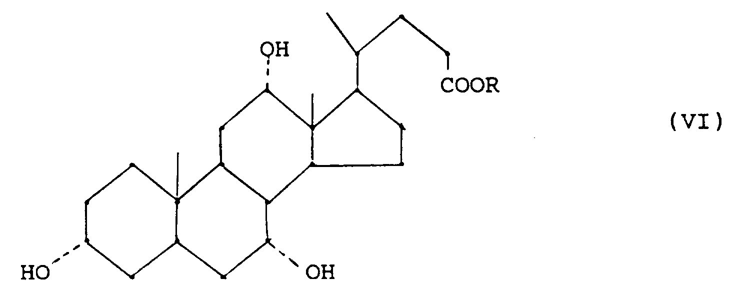 dans laquelle R est tel que défini ci-dessus, avec la quantité stoechiométrique d'anhydride acétique dans un solvant organique en présence d'une quantité catalytique d'un composé de formule (VII) 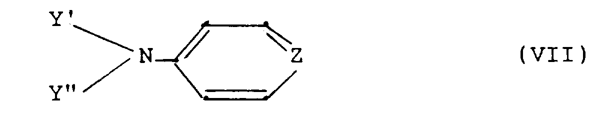 dans laquelle Y' et Y'' représentent indépendemment un alkyle de 1 à 4 atomes de carbone ou bien, ensemble, forment, avec l'atome d'azote auquel ils sont liés, un groupe pyrrolidino, pipéridino ou morpholino et Z représente un atome d'azote ou un groupe 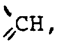 et d'une amine tertiaire en quantité inférieure à une mole d'amine par mole de produit de départ.

Le produit de départ préféré est le cholate de méthyle (formule VI, R = méthyle).

Comme catalyseur préféré on utilise la 4-diméthylaminopyridine, ci-après indiquée "4-DMAP" (formule VII, Y' = Y" = méthyle, Z = 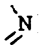 ), mais la N,N-diméthylaniline (formule VII, Y' = Y '' = méthyle, Z = 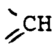 ) peut être avantageusement utilisée. La quantité de catalyseur peut varier de 0,3 à 1,5% en poids par rapport au produit (VI).

Comme solvant organique on peut utiliser un hydrocarbure aromatique tel que le benzène ou le toluène, un solvant halogéné, tel que le chlorure de méthylène ou le 1,2-dichloroéthane, un éther, tel que le méthyl-isobutyl éther ou le 1,2-diméthoxyéthane, une cétone, telle que l'acétone ou la méthylisobutyl cétone, un ester tel que l'acetate d'éthyle ou bien un mélange de deux de ces solvants. Notamment, on ajoute un deuxième solvant pour compléter la solubilisation des réactifs sans utiliser des volumes trop importants.

La réaction d'acétylation est conduite de préférence dans un mélange toluène/acétone, ou dans l'acétate d'éthyle.

L'amine tertiaire est utilisée en quantités qui peuvent varier entre 20 et 50% en poids par rapport au composé (VI). Les amines préférées sont les amines aliphatiques comme la triméthylamine ou la triéthylamine, mais des amines hétérocycliques comme la 1-méthylpipéridine ou la 1-méthylmorpholine ou des amines aromatiques comme la pyridine peuvent également être utilisées.

Cette acétylation est conduite à une température de 0-5°C, comme dans tous les cas de ce type d'acétylation.

En général, après 10-12 heures à cette température, la réaction est complète, mais sa durée n'est pas critique au delà de 12 heures car, même laissant le mélange réactionnel à 0-5°C pendant 24 heures, on n'observe aucune formation ultérieure de sous-produits. Cela constitue l'avantage principal par rapport aux procédés classiques.

Le produit final est isolé selon les techniques conventionnelles et peut être éventuellement cristallisé dans le toluène ou dans le méthanol.

Pour obtenir de bons rendements en produit (I), il est souhaitable que le produit de départ qui doit être soumis à l'acétylation soit sec, notamment qu'il ne contienne pas l'alcool utilisé pour l'estérification préalable.

On peut cependant utiliser, selon un autre aspect de la présente invention, un cholate de méthyle, d'éthyle ou de n-propyle de départ qui contient 5 à 30% de l'alcool estérifiant. Dans ce cas, on peut éliminer ledit alcool par dissolution du produit de départ dans un mélange formé par un solvant organique ayant un point d'ébullition à la pression ambiante supérieur à 100°C, de préférence le même solvant qui sera utilisé pour la réaction d'acétylation, et de l'eau et par élimination d'abord de l'eau et ensuite, azéotropiquement, du mélange solvant/eau.

Il est préférable de dissoudre le produit de départ dans le solvant qui sera utilisé pour la réaction d'acétylation et d'utiliser une quantité d'eau de 10 à 25% en volume par rapport à la quantité de solvant organique utilisé.

On peut éliminer d'abord l'eau et ensuite le mélange de l'eau et de l'alcool encore éventuellement présent par distillation azéotropique à une température supérieure à 100°C.

Selon cet aspect de la présente invention, il est donc avantageux d'utiliser le toluène comme solvant ayant un point d'ébullition à la pression ambiante supérieur à 100°C.

On a ainsi trouvé que, en ce qui concerne l'étape (b) la réaction d'oxydation des esters alkyliques de l'acide 3α, 7α-diacétoxy-12α-hydroxy-5β-cholanique (I), peut être effectuée de façon efficace et sans problème de contrôle en ajoutant lentement une quantité essentiellement stoechiométrique d'un hypochlorite alcalin à une solution du dérivé 12α-hydroxy de formule (I) et d'acide acétique en quantité stoéchiométrique, en présence d'un bromure alcalin, à une température inférieure à 40°C, et de préférence, inférieure à 10°C.

Ainsi, la préparation du 12-oxocholanate de formule (II) 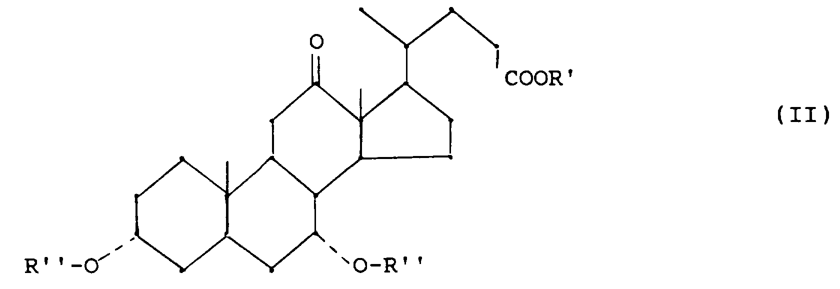 dans laquelle les deux R'' et R' représentent l'hydrogène ou bien les deux R'' représentent un groupe acétyle et R' a la même signification que R ci-dessus est plus convenablement realisée en ajoutant lentement un hypochlorite alcalin à une solution d'un 12α-hydroxy-cholanate de formule (I) 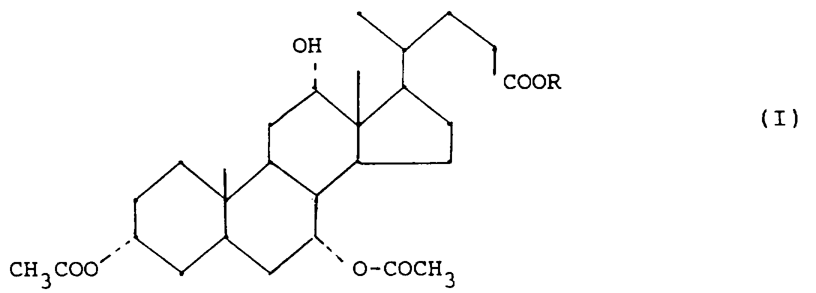 dans laquelle R est tel que défini ci-dessus, contenant de l'acide acétique en quantité stoechiométrique et un bromure alcalin, à une température inférieure à 40°C, et en saponifiant éventuellement le produit ainsi obtenu, pour préparer le composé de formule (II) dans laquelle R' et R'' représentent l'hydrogène.

Comme hydroxycholanate de départ préféré on utilise l'ester méthylique de l'acide 3α,7α-diacétoxy-12α-hydroxy-5β-cholanique.

Cette réaction peut être conduite dans un solvant organique ou organique-aqueux, le solvant organique étant un hydrocarbure halogéné, par exemple le chlorure de méthylène ou 1'1,2-dichloroéthane, l'acétate d'éthyle ou un éther, par exemple le diméthoxyéthane ou le tétrahydrofuranne.

Le bromure alcalin est de préférence utilisé à raison de 0,30-1,15 mole par mole d'hydroxycholanate de départ, le cation étant de préférence celui de l'hypochlorite utilisé. De préférence, on utilise hypochlorite de sodium et bromure de sodium.

La température de réaction doit être inférieure à 40°C pour éviter la libération de chlore, mais il est avantageux de conduire l'oxydation au dessous de 10°C et, de préférence, au dessous de 0°C.

En général, selon un mode opératoire préférentiel, la réaction est terminée après 16-24 heures à une température entre -10 et 0°C et le produit de formule (II) est isolé selon les techniques conventionelles.

Par exemple, le produit ainsi obtenu peut être isolé sous forme cristalline en versant le mélange réactionnel dans l'eau, de préférence en présence d'un sulfite alcalin qui sert à détruire l'excès d'agent oxydant encore présent, en éliminant la phase aqueuse et en évaporant le solvant.
Le 12-oxo-cholanate ainsi obtenu peut aussi être purifié en le cristallisant à partir d'un solvant ou système solvant approprié, tel que l'acétone ou les mélanges acétone-eau.

On obtient ainsi un ester de l'acide 3α,7α-diacétoxy-12-oxo-5β-cholanique qui peut être saponifié en utilisant une solution aqueuse d'hydroxyde de sodium pour isoler l'acide 3α,7α-dihydroxy-12-oxo-5β-cholanique.

En ce qui concerne la réduction d'un composé (II) pour obtenir l'acide chénodésoxycholique (III) (étape (c)), on a maintenant trouvé que, si on conduit la réaction de Wolff-Kishner dans un solvant aqueux-organique en éliminant lentement l'eau et une partie du solvant par distillation azéotropique, le déroulement de la réduction est facilement contrôlé et l'acide chénodésoxycholique est obtenu à l'état pur avec un rendement pratiquement quantitatif.

Plus particulièrement, on a trouvé que l'acide chénodésoxycholique peut être obtenu avec un bon rendement et substantiellement dépourvu d'impuretés, en particulier d'acide lithocholique, en traitant l'acide 3α,7α-dihydroxy-12-oxo-5β-cholanique ou un de ses esters de formule (II) 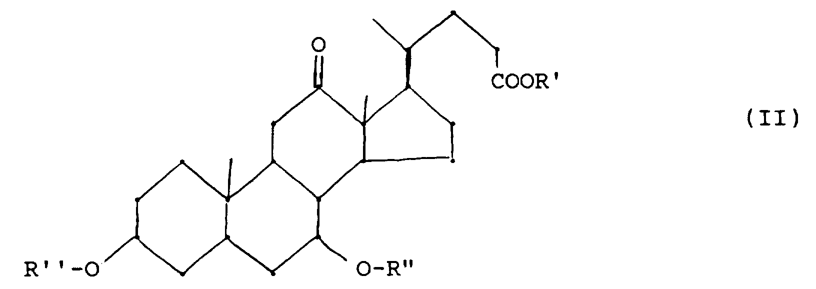 dans laquelle R' et R" représentent, l'hydrogène ou R'' représente un groupe acétyle et R' a la même signification que R ci-dessus, avec de l'hydrazine et une base minérale dans un mélange d'eau et d'un solvant organique hydroxylé qui a un point d'ébullition à pression ambiante superieur à 100°C et forme un azéotrope avec l'eau, à une température de 85-110°C, puis on distille l'eau et une partie du solvant en augmentant la température du mélange réactionnel jusqu'à 115-145°C et on laisse se terminer la réaction à cette température.

Par le terme "solvant organique hydroxylé" on entend un solvant organique choisi entre les alcanols et les éthers alkyliques inférieurs du glycol éthylénique ou propylénique.

Des solvants organiques appropriés comprennent le 2-méthoxyéthanol (nom commercial: METHYL CELLOSOLVE) le 2-éthoxyéthanol, le 2-butoxyéthanol, le 2-méthoxy-propanol, le 1-butanol et le 2-pentanol, le 2-méthoxyéthanol et le 1-butanol étant préférés.

La quantité d'eau présente dans le mélange réactionnel avec le solvant organique hydroxylé peut varier entre 10% et 30% en volume et est de préférence présente dans un pourcentage d'environ 20% en volume par rapport au solvant organique.

L'utilisation de ce système solvant permet, aux conditions prévues pour cette réaction, l'élimination lente et en même temps complète de l'eau par distillation azéotropique en permettant ainsi de poursuivre la réaction jusqu'au bout.

Le composé de départ préféré est celui de formule (II) ci-dessus, où R' = R" = H, mais 1' ester méthylique du diacétate (formule (II), R' = méthyle, R" = acétyle) convient également,

Comme, de toute façon, le produit final souhaité est l'acide chénodésoxycholique (III), la quantité de base minérale doit être calculée de façon à provoquer la saponification de tous les esters éventuellement présents.

Comme base minérale on utilise de préférence l'hydroxyde de potassium (80-85%), et l'hydrazine est également utilisée, sous forme de base, en solution aqueuse à 80-85%.

La réaction est amorcée lentement par chauffage jusqu'à la température de 85-110°C en environ 45-75 minutes, puis le mélange réactionnel est laissé à la même température pour une période de temps variable entre 1 et 5 heures.

Pour compléter la réaction, on distille le mélange réactionnel en augmentant lentement la température du mélange jusqu'à 115-145°C, puis on laisse le mélange réagir à la même temperature.

Normalement, après 5-10 heures de chauffage, la réaction est terminée et l'acide chénodésoxycholique (III) est isolé par simple acidification et filtration.

Un premier objet de la présente invention est donc un procédé de préparation de l'acide chénodésoxycholique se déroulant essentiellement selon les étapes suivantes:
- **étape (a)** - 3,7-diacétylation du cholate de méthyle, éthyle ou n-propyle pour obtenir l'ester correspondant de formule (I) 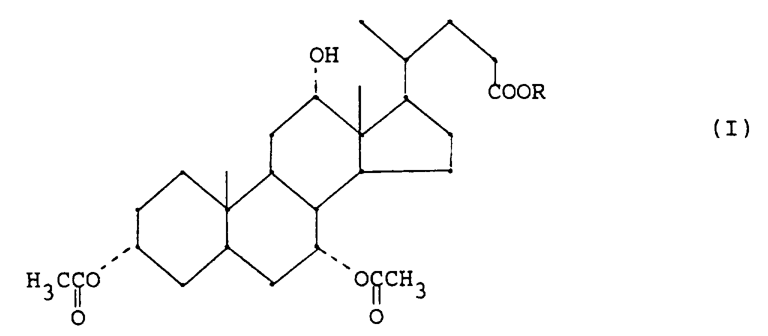 dans laquelle R est méthyle, éthyle, ou n-propyle,
- **étape (b)** - oxydation d'un composé de formule (I) en dérivé 12-oxo de formule (II) 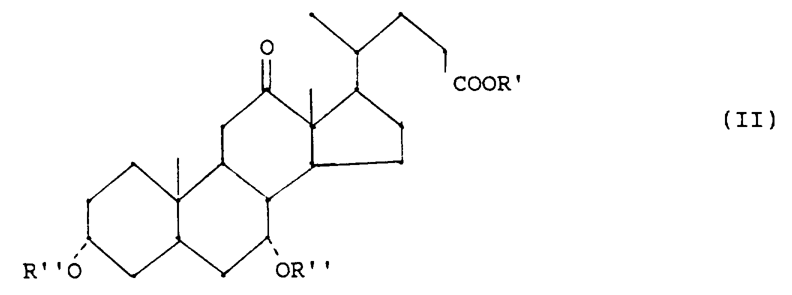 dans laquelle R' et R'' représentent l'hydrogène ou bien R'' représente un groupe acétyle et R' a la même signification que R ci-dessus, et
- **étape (c)** - réduction d'un composé de formule (II) en acide chénodésoxycholique de formule (III) 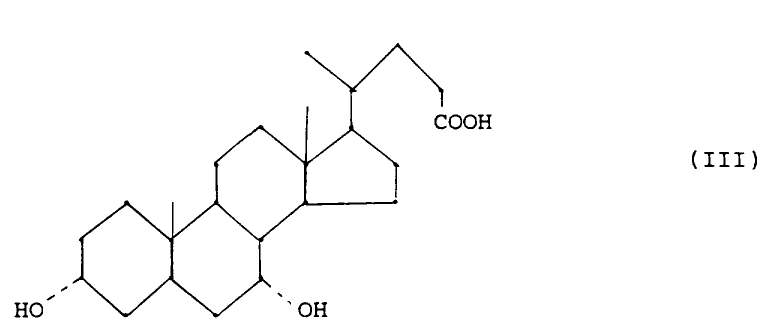 caractérisé en ce que
   - (a) : on prépare un composé de formule (I) en traitant un ester de l'acide cholique de fomule (VI) 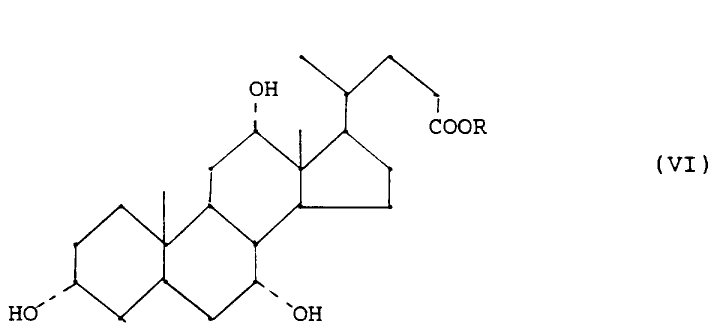 dans laquelle R est méthyle, éthyle ou n-propyle, avec la quantité stoechiométrique d'anhydride acétique dans un solvant organique en présence d'une quantité catalytique d'un composé de formule (VII) 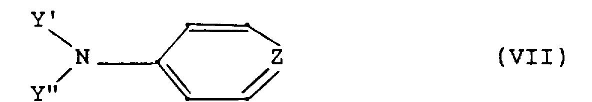 dans laquelle Y' et Y'' représentent indépendamment un groupe (C₁ - C₄) alkyle ou bien, ensemble, forment, avec l'atome d'azote auquel ils sont liés, un groupe pyrrolidino, pipéridino ou morpholino et Z représente un atome d'azote ou un groupe 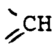 , et d'une amine tertiaire en quantité inférieure à une mole d'amine par mole de produit de départ;
   - (b): on oxyde un composé de formule (I) en dérivé 12-oxo de formule (II) en ajoutant lentement un hypochlorite alcalin en quantité essentiellement stoechiométrique à une solution d'un 12α-hydroxycholanate de formule (I) dans laquelle R est tel que defini ci-dessus et d'acide acétique en quantité stoechiométrique, en présence d'un bromure alcalin, à une température inferieure à 40°C, et en saponifiant éventuellement le produit ainsi obtenu pour obtenir le composé de formule (II) dans laquelle R' et R'' sont l'hydrogène; et
   - (c): on réduit un composé de formule (II) en acide chénodésoxycholique (III) en traitant ledit composé de formule (II) dans laquelle R' et R'' sont tels que definis ci-dessus, avec de l'hydrazine et une base minérale dans un mélange formé par de l'eau et un solvant organique hydroxylé ayant un point d'ébullition à pression ambiante supérieur à 100°C et formant un azéotrope avec l'eau, à une température de 85-110°C, puis on distille l'eau et une partie du solvant en augmentant la température du mélange réactionnel jusqu'à 115-145°C et on laisse terminer la réaction à cette température.

Il est entendu que toutes les indications concernant les conditions de réaction encore plus avantageuses qui ont été mentionnées ci-dessus pour chaque étape, s'appliquent également au procédé global de préparation de l'acide chénodésoxycholique et représentent des formes de réalisation préférés dudit procédé.

Les exemples suivants illustrent la présente invention sans toutefois la limiter.

### Etape (a) - Préparation des esters alkyliques de l'acide 3α,7α-diacétoxy-l2α-hydroxy-5β-cholanique (I)

### Exemple 1

On chauffe à 40°C une mélange de 105 kg de cholate de méthyle provenant directement de l'estérification de l'acide cholique avec le méthanol et contenant 15% de méthanol, 580 l de toluène et 90 l d'eau; puis on sépare la phase aqueuse, on chauffe la phase organique au reflux et on élimine l'eau azéotropiquement jusqu'à 110°C. On refroidit, on ajoute 135 l d'acétone et, à une température inférieure à 20°C, on ajoute d'abord 0,78 kg de 4-DMAP et 35 kg de triéthylamine et ensuite, lentement, 44 kg d'anhydride acétique. On porte la température à 0-5°C et on laisse le mélange réactionnel à cette température jusqu'à ce que la réaction est complète. Ensuite, on lave avec 180 l d'eau et on concentre la phase organique jusqu'à une concentration de 1:2. On amorce la cristallisation à 60°C, on isole le produit à 0-5°C par centrifugation et on le lave avec du toluène et de l'eau. Après une cristallisation dans le toluène, on obtient le 3α,7α-diacétoxy-12α-hydroxy-5β-cholanate de méthyle, identique à un échantillon authentique, ayant un titre supérieur à 99% mesuré par chromatographie en phase gazeuse. Rendement en produit cristallisé: 76% sur 90 kg estimés de produit de départ sec.

### Exemple 2

En opérant comme décrit dans l'Exemple 1, mais en remplaçant les 35 kg de triéthylamine par 35 kg de pyridine on obtient le 3α,7α-diacétoxy-12α-hydroxy-5β-cholanate de méthyle ayant le même dégré de pureté et avec le même rendement.

### Etape (b) Oxydation d'un composé (I) en derivé 12-oxo (II)

### Exemple 3

A une solution de 17 kg de 3α,7α-diacétoxy-12α-hydroxy-5β-cholanate de méthyle et 1,04 kg de bromure de sodium dans 46 kg d'acétate d'éthyle, 3,6 kg d'acide acétique et 3,4 kg d'eau, refroidie à -10°C-0°C, on ajoute en 5 heures 18,48 l d'une solution à 14,9 % poids/volume d'hypochlorite de sodium. On laisse le mélange réactionnel 16 heures à la même température, puis on ajoute 17 kg d'une solution de sulfite de sodium à 10% et on chauffe à 40°C. On élimine la phase aqueuse, on lave à une solution de bicarbonate de sodium jusqu'à neutralité, et on ajoute 1,1 volumes d'eau par volume de 3α,7α-diacétoxy-12α-hydroxy-5β-cholanate de méthyle. On distille à 100°C, et on refroidit la suspension à une température inférieure à 50°C. On y ajoute 3,1 volumes d'acetone et on chauffe au reflux pendant une heure jusqu'à l'obtention d'une solution. On refroidit et on laisse cristalliser. On abaisse la température jusqu'à 0-5°C pendant 4 heures et on centrifuge en lavant le produit cristallisé avec un mélange glacé eau-acétone 1:1. On obtient 15,57 kg de 3α,7α-diacétoxy-12-oxo-5β-cholanate de méthyle ayant un titre supérieur à 99% par rapport à un échantillon authentique. On peut aussi, après neutralisation de la solution du produit dans l'acétate d'éthyle, évaporer le solvant sur un bain d'eau, refroidir et filtrer en obtenant le3α,7α-diacétoxy-12-oxo-5β-cholanate de méthyle avec un rendement quantitatif.

### Exemple 4

On opère comme décrit ci-dessus jusqu'à la cristallisation du 3α,7α-diacétoxy-12-oxo-5β-cholanate de méthyle, puis on ajoute 4 équivalents d'hydroxyde de sodium à 40% et on chauffe au reflux jusqu'à ce que la saponification soit complète. Par acidification avec de l'acide chlorhydrique, on obtient l'acide 3α,7α-dihydroxy-12-oxo-5β-cholanique ayant un titre supérieur à 99% par rapport à un échantillon authentique.

### Etape (c) Réduction d'un composé (II) en acide chénodésoxy-cholique

### Exemple 5

A un mélange de 40 g d'acide 3α,7α-dihydroxy-12-oxo-5β-cholanique, 80 ml de METHYL CELLOSOLVE et 17 ml d'eau, on ajoute 40 g d'hydroxyde de potassium à 85% et, ensuite, 13 ml d'hydrazine à 80%. On porte la température à 110°C et on continue le chauffage à la même température pendant 4 heures. Ensuite, on distille le mélange réactionnel jusqu'à la température de 135-138°C et on le laisse à la même température pendant 8 heures. On refroidit, on ajoute 120 ml d'eau, puis on verse la solution dans 160 ml d'eau contenant 40 ml d'acide sulfurique 50 B à une température inférieure à 20°C en ajustant le pH à environ 2. On filtre et on lave jusqu'à neutralité. On obtient ainsi 40 g d'acide chénodésoxycholique pur ayant un titre de 86,5 %, les impuretés majeures étant l'eau et le sulfate de potassium. La quantité d'acide lithocholique, évaluée par chromatographie sur couche mince est << 0.05%

### Exemple 6

On chauffe un mélange de 50g d'ester méthylique de l'acide 3α,7α-diacétoxy-12-oxo-5β-cholanique, 250 ml d'alcool n-butylique et 25 g d'hydrate d'hydrazine à 80% à 103°C et on continue le chauffage à la même température pendant 2 heures. Ensuite on distille le mélange réactionnel jusqu'à la température de 117-122°C, on ajoute 50 g d'hydroxyde de potassium à 88% et on distille le mélange réactionnel jusqu'à la température de 138-142°C. On continue le chauffage à la même température pendant 24 heures. On refroidit, on ajoute 200 ml d'eau, on distille le mélange jusqu'à l'élimination totale de l'alcool n-butylique, on refroidit, on ajoute 130 ml d'eau et d'acide formique à 60% jusqu'à pH 7-8, on ajoute 100 ml d'acétate d'éthyle et d'acide formique jusqu'à pH 4-5. On separe la phase organique qui est lavée avec une solution aqueuse de bicarbonate de sodium jusqu'à neutralité, on separe la phase organique; le produit est extrait par une solution aqueuse de bicarbonate de sodium.
La phase aqueuse est séparée et on distille jusqu'à l'élimination totale de l'acétate d'éthyle.
On refroidit et on verse la solution dans une quantité d'acide sulfurique 20% pour obtenir un pH final d'environ 2, on filtre et on lave jusqu'à neutralité. On obtient ainsi 36,8 g d'acide chénodésoxycholique pur ayant un titre de 97,2%, l'impureté majeure étant l'eau.

### Exemple 7

On opère substantiellement comme décrit dans l'exemple 6 en utilisant le méthyl cellosolve au lieu de l'alcool n-butylique. On chauffe pendant 10 heures au lieu de 24 et on isole l'acide chénodésoxycholique selon la méthode décrite dans l'exemple 5. On obtient 38,1 g d'acide chénodésoxycholique pur ayant un titre de 98,5%, l'impureté majeure étant l'eau.

## Revendications

1. Procédé de préparation de l'acide chénodésoxycholique se déroulant selon les étapes suivantes :
- **étape (a)** : 3,7-diacétylation du cholate de méthyle, éthyle ou n-propyle pour obtenir l'ester correspondant de formule (I) 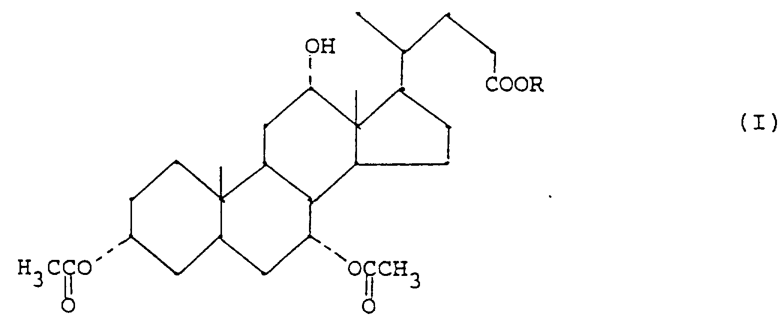 dans laquelle R est méthyle, éthyle, ou n-propyle,
- **étape (b)** : oxydation d'un composé de formule (I) en dérivé 12-oxo de formule (II) 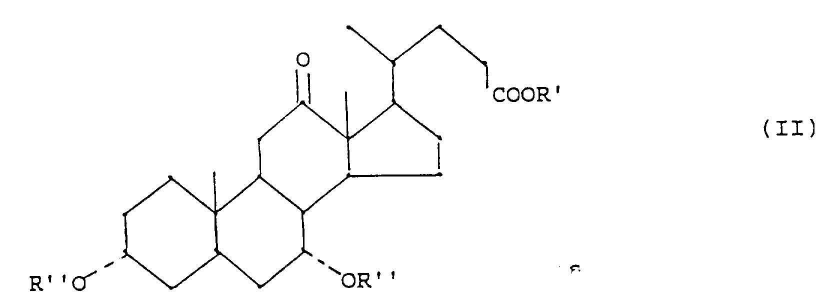 dans laquelle R' et R'' représentent l'hydrogène ou bien R" représente un groupe acétyle et R' a la même signification que R ci-dessus, et
- **étape (c)** : réduction d'un composé de formule (II) en acide chénodésoxycholique de formule (III) 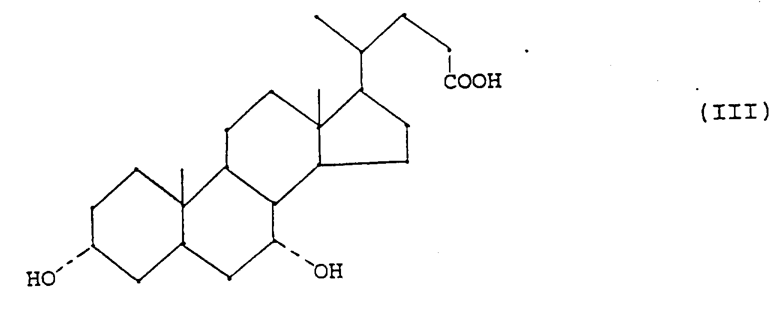 caractérisé en ce que :
(a) : on prépare un composé de formule (I) telle que définie ci-dessus en traitant un ester de l'acide cholique de formule (VI) 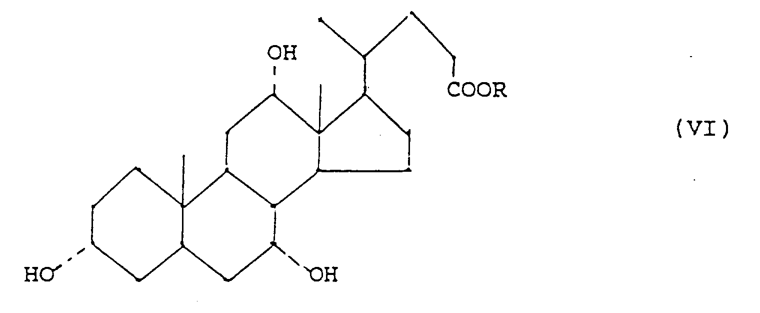 dans laquelle R est tel que défini ci-dessus avec une quantité stoechiométrique d'anhydride acétique dans un solvant organique en présence d'une quantité catalytique d'un composé de formule (VII) 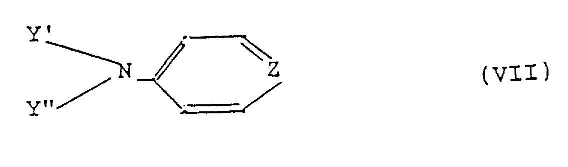 dans laquelle Y' et Y'' représentent indépendamment un groupe (C₁-C₄) alkyle ou bien, ensemble, forment, avec l'atome d'azote auquel ils sont liés, un groupe pyrrolidino, pipéridino ou morpholino et Z représente un atome d'azote ou un groupe 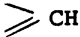 , et d'une amine tertiaire en quantité inférieure à une mole d'amine par mole de produit de départ;
(b) : on oxyde un composé de formule (I) en dérivé 12-oxo de formule (II) en ajoutant lentement un hypochlorite alcalin à une solution de 12α-hydroxycholanate de formule (I) dans laquelle R est tel que défini ci-dessus et d'acide acétique en quantité stoechiométrique, en présence d'un bromure alcalin, à une température inférieure à 40°C et en saponifiant éventuellement le produit ainsi obtenu pour obtenir le composé de formule (II) dans laquelle R' et R'' sont l'hydrogène;
(c) : on réduit un composé de formule (II) en acide chénodésoxycholique (III) en traitant ledit composé de formule (II) dans laquelle R' et R'' sont tels que définis ci-dessus avec de l'hydrazine et une base minérale dans un mélange formé par de l'eau et un solvant organique hydroxylé ayant un point d'ébullition à pression ambiante supérieur à 100°C et formant un azéotrope avec l'eau, à une température de 85-110°C, puis on distille l'eau et une partie du solvant en augmentant la température du mélange réactionnel jusqu'à 115-145°C et on laisse se terminer la réaction à cette température.

2. Procédé selon la revendication 1, caractérisé en ce que comme composé de départ dans l'étape (a) on utilise un cholate de méthyle, d'éthyle ou de n-propyle qui contient 5 à 30% de l'alcool estérifiant et qu'on élimine ledit alcool par dissolution dans un mélange formé par un solvant organique ayant un point d'ébullition à la pression ambiante supérieur à 100°C et de l'eau et par élimination d'abord de l'eau et ensuite, azéotropiquement, du mélange solvant/eau.

3. Procédé selon l'une des revendications 1 ou 2, caractérisé en ce que comme produit de départ dans l'étape (a) on utilise le cholate de méthyle et comme solvant organique le toluène.

4. Procédé selon l'une quelconque des revendications 1 à 3, caractérisé en ce que dans l'étape (a) on utilise comme catalyseur la 4-diméthylaminopyridine.

5. Procédé selon l'une quelconque des revendications 1, 2 ou 4, caractérisé en ce que l'étape (a) est conduite dans un mélange toluène/acétone, ou dans l'acétate d'éthyle.

6. Procédé selon la revendication 1, caractérisé en ce que dans l'étape (b) on utilise le 3α,7α-diacétoxy-12α-hydroxy-5β-cholanate de méthyle en tant que produit de départ.

7. Procédé selon la revendication 6, caractérisé en ce que dans l'étape (b) le bromure alcalin est utilisé à raison de 0,30 - 1, 15 mole par mole d'hydroxycholanate de départ.

8. Procédé selon l'une quelconque des revendications 6 ou 7, caractérisé en ce que dans l'étape (b) on utilise l'hypochlorite de sodium et le bromure de sodium.

9. Procédé selon la revendication 1, caractérisé en ce que dans l'étape (c) l'eau est présente dans le mélange réactionnel dans une quantité de 10 à 30% en volume par rapport au solvant organique hydroxylé.

10. Procédé selon la revendication 9, caractérisé en ce que dans l'étape (c) on utilise le 2-méthoxyéthanol ou le 1-butanol en tant que solvant organique hydroxylé.

11. Procédé selon l'une quelconque des revendications 9 ou 10, caractérisé en ce que dans l'étape (c) on utilise l'acide 3α, 7α-dihydroxy-12-oxo-5 β-cholanique libre en tant que produit de départ.

## Claims

1. Process for the preparation of chenodeoxycholic acid, which takes place according to the following stages:
- **stage (a)**: 3,7-diacetylation of methyl, ethyl or n-propyl cholate in order to obtain the corresponding ester of formula (I) 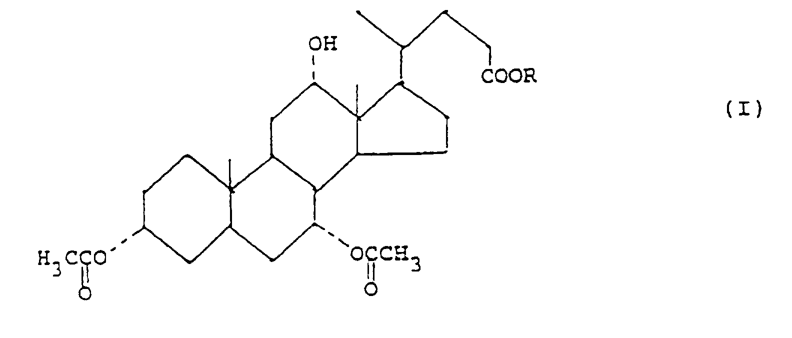 in which R is methyl, ethyl or n-propyl,
- **stage (b):** oxidation of a compound of formula (I) to the 12-oxo derivative of formula (II) 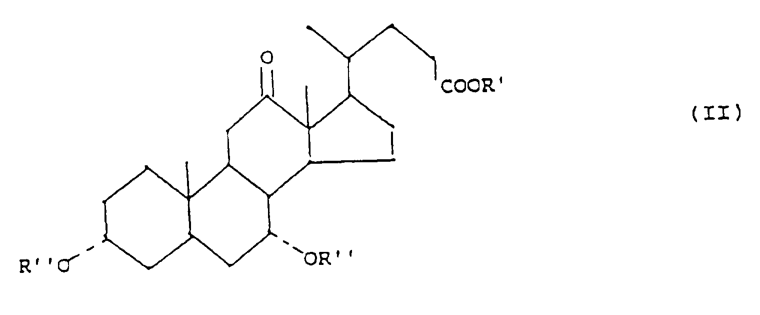 in which R' and R" represent hydrogen or else R'' represents an acetyl group and R' has the same meaning as R above, and
- **stage (c)**: reduction of a compound of formula (II) to chenodeoxycholic acid of formula (III) 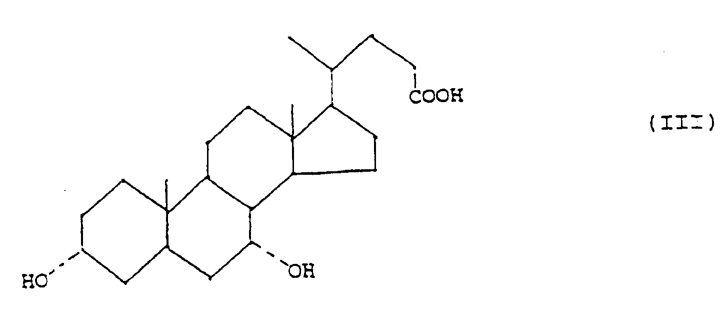 characterized in that :
(a): a compound of formula (I) as defined above is prepared by treating an ester of cholic acid of formula (VI) 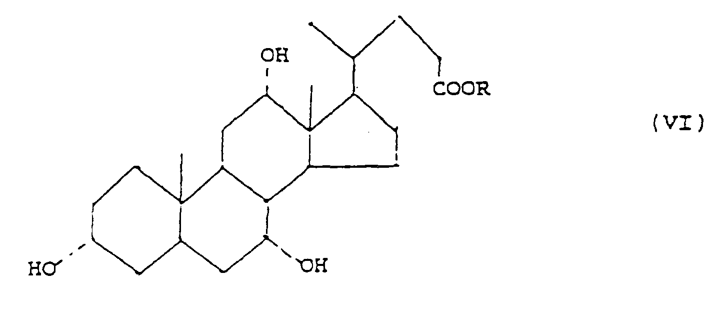 in which R is as defined above, with a stoichiometric amount of acetic anhydride in an organic solvent in the presence of a catalytic amount of a compound of formula (VII) 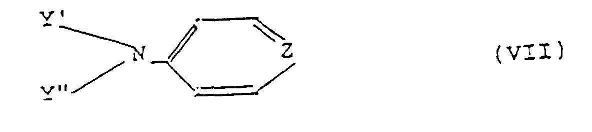 in which Y' and Y'' independently represent a (C₁-C₄) alkyl group or else, together, form, with the nitrogen atom to which they are bonded, a pyrrolidino, piperidino or morpholino group and Z represents a nitrogen atom or a 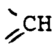 group, and of a tertiary amine in an amount of less than one mole of amine per mole of starting material;
(b): a compound of formula (I) is oxidized to the 12-oxo derivative of formula (II) by slowly adding an alkali metal hypochlorite to a solution of 12α-hydroxycholanate of formula (I), in which R is as defined above, and of acetic acid in a stoichiometric amount, in the presence of an alkali metal bromide, at a temperature of less than 40°C, and by optionally saponifying the product thus obtained in order to obtain the compound of formula (II) in which R' and R'' are hydrogen;
(c) : a compound of formula (II) is reduced to chenodeoxycholic acid (III) by treating the said compound of formula (II), in which R' and R'' are as defined above, with hydrazine and an inorganic base in a mixture formed by water and a hydroxylated organic solvent, which has a boiling point at ambient pressure greater than 100°C and which forms an azeotrope with water, at a temperature of 85-110°C, the water and part of the solvent are then distilled off by increasing the temperature of the reaction mixture to 115-145°C and the reaction is left to come to completion at this temperature.

2. Process according to Claim 1, characterized in that a methyl, ethyl or n-propyl cholate which contains 5 to 30 % of the esterifying alcohol is used as starting compound in stage (a) and in that the said alcohol is removed by dissolving in a mixture formed by an organic solvent, which has a boiling point at ambient pressure greater than 100°C, and water and by removal first of water and then, azeotropically, of the solvent/water mixture.

3. Process according to either of Claims 1 and 2, characterized in that methyl cholate is used as starting material in stage (a) and toluene is used as organic solvent.

4. Process according to any one of Claims 1 to 3, characterized in that 4-dimethylaminopyridine is used as catalyst in stage (a).

5. Process according to any one of Claims 1, 2 and 4, characterized in that stage (a) is carried out in a toluene/acetone mixture or in ethyl acetate.

6. Process according to Claim 1, characterized in that, in stage (b), methyl 3α,7α-diacetoxy-12α-hydroxy-5β-cholanate is used as starting material.

7. Process according to Claim 6, characterized in that, in stage (b), the alkali metal bromide is used in a proportion of 0.30 - 1.15 mol per mole of starting hydroxycholanate.

8. Process according to either of Claims 6 and 7, characterized in that, in stage (b), sodium hypochlorite and sodium bromide are used.

9. Process according to Claim 1, characterized in that, in stage (c), the water is present in the reaction mixture in an amount of 10 to 30 % by volume with respect to the hydroxylated organic solvent.

10. Process according to Claim 9, characterized in that, in stage (c), 2-methoxyethanol or 1-butanol is used as hydroxylated organic solvent.

11. Process according to either of Claims 9 and 10, characterized in that, in stage (c), free 3α,7α-dihydroxy-12-oxo-5β-cholanic acid is used as starting material.

## Patentansprüche

1. Verfahren zur Herstellung von Chenodesoxycholsäure, das folgende Stufen umfaßt:
- **Stufe (a)**: 3,7-Diacetylierung von Methyl-, Ethyl- oder n-Propylcholat unter Erhalt des entsprechenden Esters der Formel (I), 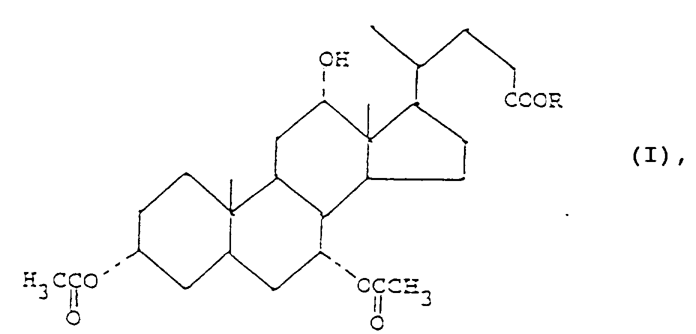 in der R Methyl, Ethyl oder n-Propyl bedeutet,
- **Stufe (b)**: Oxidation einer Verbindung der Formel (I) zum 12-Oxoderivat der Formel (II), 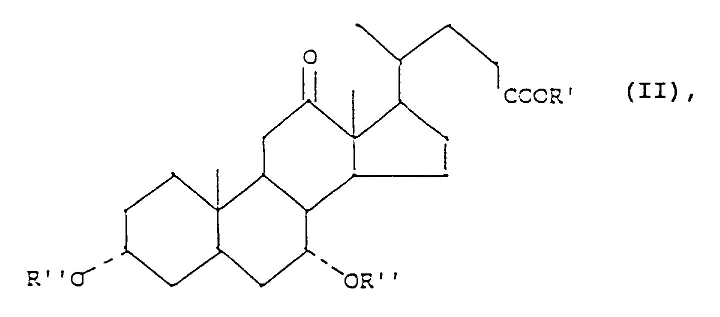 in der bedeuten:
R' und R" Wasserstoff, oder R" Acetyl und R" dasselbe wie oben für R definiert, und
- **Stufe (c)**: Reduktion einer Verbindung der Formel (II) zu Chenodesoxycholsäure der Formel (III), 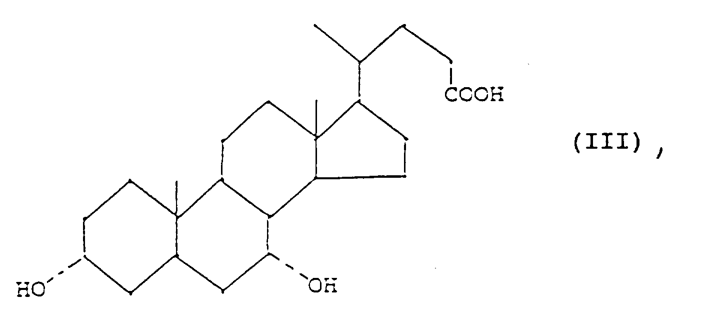 gekennzeichnet durch folgende Maßnahmen:
**Stufe (a)**: Herstellung einer Verbindung der Formel (I) wie oben definiert durch Behandlung eines Cholsäureesters der Formel (VI), 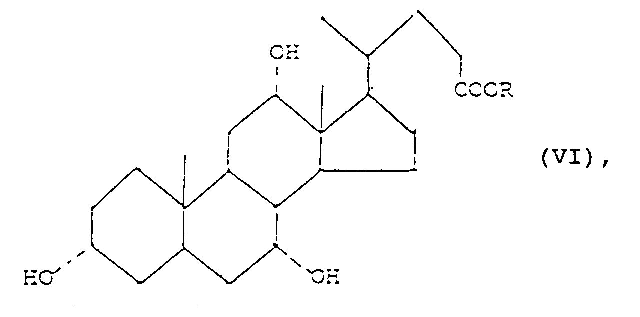 in der R wie oben definiert ist,
mit einer stöchiometrischen Menge Acetanhydrid in einem organischen Lösungsmittel in Gegenwart einer katalytischen Menge einer Verbindung der Formel (VII), 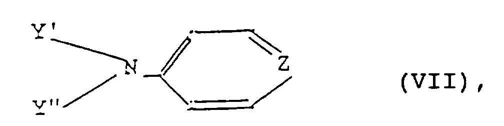 in der
Y' und Y'' unabhängig C₁₋₄-Alkyl bedeuten oder zusammen mit dem Stickstoffatom, an dem sie gebunden sind, eine Pyrrolidino-, Piperidino- oder Morpholinogruppe darstellen und
Z ein Stickstoffatom oder eine Gruppe 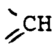 bedeutet,
sowie einem tertiären Amin in einer Menge, die kleiner ist als 1 mol Amin pro Mol Ausgangsprodukt;
**Stufe (b)**: Oxidation einer Verbindung der Formel (I) zum 12-Oxoderivat der Formel (II) durch langsame Zugabe eines Alkalihypochlorits zu einer Lösung des 12-α-Hydroxycholanats der Formel (I), in der R wie oben definiert ist, sowie von Essigsäure in stöchiometrischer Menge in Gegenwart eines Alkalibromids bei einer Temperatur unter 40 °C sowie ggfs. Verseifung des so erhaltenen Produkts unter Erhalt der Verbindung der Formel (II), in der R' und R'' Wasserstoff bedeuten;
**Stufe (c)**: Reduktion einer Verbindung der Formel (II) zu Chenodesoxycholsäure der Formel (III) durch Behandlung der Verbindung der Formel (II), in der R' und R'' wie oben definiert sind, mit Hydrazin und einer anorganischen Base in einem Gemisch aus Wasser und einem organischen, hydroxylhaltigen Lösungsmittel mit einem Siedepunkt bei einem Umgebungsdruck von mehr als 100 °C, das mit Wasser ein Azeotrop bildet, bei einer Temperatur von 85 bis 110 °C, anschließendes Abdestillieren des Wassers und eines Teils des Lösungsmittels durch Erhöhung der Temperatur des Reaktionsgemisches bis auf 115 bis 145 °C und Endenlassen der Umsetzung bei dieser Temperatur.

2. Verfahren nach Anspruch 1, dadurch gekennzeichnet, daß als Ausgangsverbindung in Stufe (a) ein Methyl-, Ethyl- oder n-Propylcholat eingesetzt wird, das 5 bis 30 % des zur Veresterung verwendeten Alkohols enthält, und dieser Alkohol durch Lösen in einem Gemisch aus einem organischen Lösungsmittel mit einem Siedepunkt bei Umgebungsdruck, der höher als 100 °C ist, und Wasser sowie zunächst Abtrennung des Wassers und anschließende Abtrennung des Lösungsmittel-Wasser-Gemischs als Azeotrop abgetrennt wird.

3. Verfahren nach Anspruch 1 oder 2, dadurch gekennzeichnet, daß als Ausgangsprodukt in Stufe (a) Methylcholat und als organisches Lösungsmittel Toluol eingesetzt werden.

4. Verfahren nach einem der Ansprüche 1 bis 3, dadurch gekennzeichnet, daß in Stufe (a) als Katalysator 4-Dimethylaminopyridin eingesetzt wird.

5. Verfahren nach Anspruch 1, 2 oder 4, dadurch gekennzeichnet, daß Stufe (a) in einem Toluol-Aceton-Gemisch oder in Ethylacetat durchgeführt wird.

6. Verfahren nach Anspruch 1, dadurch gekennzeichnet, daß in Stufe (b) als Ausgangsprodukt 3α-7α-Diacetoxy-12α-hydroxy-5β-cholansäuremethylester eingesetzt wird.

7. Verfahren nach Anspruch 6, dadurch gekennzeichnet, daß in Stufe (b) das Alkalibromid in einer Menge von 0,30 bis 1,15 mol pro Mol des als Ausgangsverbindung eingesetzten Hydroxycholanats eingesetzt wird.

8. Verfahren nach Anspruch 6 oder 7, dadurch gekennzeichnet, daß in Stufe (b) Natriumhypochlorit und Natriumbromid eingesetzt werden.

9. Verfahren nach Anspruch 1, dadurch gekennzeichnet, daß in Stufe (c) Wasser im Reaktionsgemisch in einer Menge von 10 bis 30 Vol.-%, bezogen auf das hydroxylhaltige organische Lösungsmittel, vorliegt.

10. Verfahren nach Anspruch 9, dadurch gekennzeichnet, daß in Stufe (c) als hydroxylhaltiges organisches Lösungsmittel 2-Methoxyethanol oder 1-Butanol eingesetzt werden.

11. Verfahren nach Anspruch 9 oder 10, dadurch gekennzeichnet, daß in Stufe (c) als Ausgangsprodukt freie 3α,7α-Dihydroxy-12-oxo-5β-cholansäure eingesetzt wird.
